# EUROPEAN PATENT SPECIFICATION

(11) **EP 3 622 964 B1**
(45) Date of publication and mention of the grant of the patent: **30.03.2022**
(21) Application number: 19195757.0
(22) Date of filing: 05.09.2019
(51) Int. Cl.: A61K 38/08, A61P 17/02

(54) **AGENT FOR THE TREATMENT OF SKIN WOUNDS OR BURNS**
MITTEL ZUR BEHANDLUNG VON HAUTWUNDEN ODER VERBRENNUNGEN
AGENT POUR LE TRAITEMENT DES PLAIES OU DES BRÛLURES DE LA PEAU

(30) Priority: 12.09.2018 RU 2018132558
(43) Date of publication of application: 18.03.2020
(73) Proprietor: PVP Labs PTE. Ltd., The Adelphi, Singapore 179803 (SG)
(72) Inventor: Chertorizhsky, Evgeny Alexandrovich, 249007 Belkino Village, Borovsky District, Kaluga Region (RU); Ovchinnikov, Mikhail Vladimirovich, 115162 Moscow (RU); Kleimenov, Aleksey Viktorovich, 249192 Zhukov, Kaluga Region (RU)
(74) Representative: Spengler, Robert

(56) References cited:
- EP-A1- 3 556 377
- Ob. Ilinski ET AL: "A new peptide activator of reparative tissue regeneration", Farmakol Toksikol, 1 July 1987 (1987-07-01), pages 64-66, XP55629618, Retrieved from the Internet: URL:https://www.ncbi.nlm.nih.gov/pubmed/33 11802 [retrieved on 2019-10-08]
- DATABASE CAPLUS, CHEMICAL ABSTRACT [Online] 1 January 1988 (1988-01-01), SHEKHTER A B ET AL: "Effect of dalargin on repair processes in wound healing", XP002682920, retrieved from CAPLUS; STN Database accession no. 1988:622915

## Description

### Pertinent art

The invention pertains to medicine and concerns the application of the hexapeptide tyrosyl-D-alanyl-glycyl-phenylalanyl-leucyl-arginine for the treatment of skin wounds or burns as a medicinal product for topical use.

### State of the art

According to WHO, over 180 thousand people die of thermal injury every year. Meanwhile, burns are a major cause of long-term hospitalization and persistent disability, and they are often accompanied by social stigma and significantly degrade the quality of life [1]. The principle of unity of topical and systemic therapy that underpins the treatment of burn patients places special emphasis on topical treatment, which is aimed at thorough healing of burn wounds and early restoration of the skin [2]. The development of new effective topical burn treatments therefore remains an important objective for public health.

Thermal injury is associated with an entire complex of pathophysiological mechanisms, such as hypoxia and lipid peroxidation (LPO), reduction of tissue perfusion in the zone of stasis, neutrophil aggregation and hyperactivation, a cascade of inflammatory reactions and severe pain. New products must obviously rely on a multifaceted mechanism of action, which is aligned with the pathogenetic aspects of injury. From this perspective, clinical development of a new burn treatment agent on the basis of a synthetic regulatory peptide with a wide spectrum of biological activity represents considerable interest.

As a component of the stress-limiting system, this hexapeptide exhibits antioxidant properties [3], inhibits LPO [4] and improves peripheral microcirculation [5].

Despite being a mediator of the opioid antinociceptive system, it is almost completely incapable of crossing the blood-brain barrier. It predominantly exhibits its analgesic effect at the peripheral/tissue level, which makes it possible to consider this product as having some properties of a topical anaesthetic [6].

Additionally, this hexapeptide also showed serious promise with respect to tissue regeneration. Specifically, several researchers demonstrated that it stimulated mitotic activity in cells by interacting with their opioid receptors and activated wound repair mechanisms [7, 8, 9]. Further, Ob. llinski et al describe in "A new peptide activator of reparative tissue regeneration", Farmakol Toksikol, 1 July 1987, pages 64-66 that synthetic analog of leucine enkephalin--hexapeptide dalargin was shown to stimulate healing of skin wounds in rats, dogs and mini-pigs at local and parenteral administration in doses of 0.1-100 micrograms/kg.

This hexapeptide in the form of the pharmaceutical drug Dalargin was authorised for human use in Russia for the treatment of peptic ulcer disease and pancreatitis as a product reducing the acidity of gastric juice and exocrine activity of the pancreas [10]. However, the efficacy of this drug in the therapy of peptic ulcer disease is also largely due to its reparative effect on gastric and duodenal mucosa [11, 12, 13]. The efficacy of Dalargin as part of combination therapy of Crohn's disease (IM 1 mg twice a day) (patent RU2363455, application No. 2007142745/14 dated 19/11/2007) is also suggestive of a systemic reparative effect. The work of E.V. Maksakova suggested that the wound healing potential of Dalargin should be used in the treatment of corneal injuries [14].

The "decision-making process" happening at the cellular level (apoptosis or DNA synthesis and mitosis) largely depends on oxidative status or redox status [15], which is especially important for reducing the zone of stasis of burn wounds. Antioxidant properties, antiradical and nitrergic activity [16] obviously give Dalargin additional capabilities related to regeneration in burn wounds.

An invention in the field of cosmetology was published, in which Dalargin was used as a component of an anti-inflammatory cream for alleviating the symptoms of acne (RU 2.045.949). Another patent (RU 2221547, application No. 2002134728/15 dated 24/12/2002) claimed immunomodulating activity of Dalargin as a component of a cosmetic product. Further, EP 3 556 377 A1 describes the use of the hexapeptide tyrosyl-D-alanyl-phenylalanyl-leucylarginine as antiviral immunotropic agent for the treatment of acute respiratory viral infections.

Data of an experiment are available, which demonstrated in vitro that Dalargin, when added to peripheral blood samples of burn patients (n = 15), normalised elevated functional and metabolic activity of neutrophils, reduced elevated activity of NADPH oxidase, alkaline phosphatase (ALP), myeloperoxidase and nuclear chromatin and normalised elevated levels of cationic proteins in neutrophils and the total number of activated neutrophils. Importantly, in the blood of healthy humans (n = 27), Dalargin had the opposite effect of increasing the total number of activated neutrophils and NADPHase activity. These effects were mediated by opioid receptors and were dose-dependent, with maximum effect being achieved at a molar concentration of 5.10⁻⁸ M [17]. However, this effect was not studied in burn models in vivo.

In general, any tissue injury results in neutrophil activation, which leads to increased production of active oxygen radicals, LPO and triggers a whole cascade of reactions that damage tissues spared by the pathological process [18]. Therefore, the ability of Dalargin to normalise neutrophil hyperactivation is a pathogenetically important link in its burn healing effect.

Results of experiments investigating the efficacy of parenteral administration of Dalargin for burn treatment have been published, but burn healing activity of the drug when used as a topical application has not been addressed in any of the studies. One work compared Dalargin with delta sleep-inducing peptide (DSIP) in burn treatment; both products were administered to test animals intraperitoneally (patent No. 2070054, publication date 10/12/1996). In another study, Dalargin (0.3 mg SC) was associated with significant acceleration of skin repair and reduced intensity of productive inflammation in a 3^{rd}-degree burn model in rats [9]. Patent RU 2196603 discloses the use of Dalargin (1 - 2 mg in 1 - 2 mL by IV infusion) for the treatment of burns as part of infusion therapy.

Unlike the works cited, the present invention discloses the potential of Dalargin as a medicinal product for topical use.

Additionally, there are data concerning the use of Dalargin in a number of other diseases. Patents UA 6829U, UA 6823U and UA 6826U disclose the use of Dalargin for the treatment of acute experimental pancreatitis. The use of Dalargin as an anti-stress agent was covered in patents UA 67632, UA 67630 and UA 67629 in experimental models of chronic pancreatitis, acute adnexitis and peritonitis respectively. The anti-stress activity of Dalargin was demonstrated in patent UA 67626 in an experimental chronic stress model. Patent RU 2180598 discloses the use of Dalargin for the treatment of toxic hepatitis in patients with chronic drug addiction. The efficacy of Dalargin in the treatment of viral diseases was demonstrated in patents RU 2261722 (treatment of a latent form of genital herpes in a woman with the foetal loss syndrome) and RU 2167671 (treatment of tick-borne encephalitis). Publication MD1413F discloses the use of Dalargin for the treatment of the oral mucosa and lichen planus, and publication MD1296F presents data on the therapy of lichen planus with Dalargin. Patent RU 2008131509 presents a pharmaceutical composition for the treatment of demyelinating diseases, which includes Dalargin. Patent RU 2218896 discloses the use of Dalargin for the treatment of bullous keratopathy. Publications MD1963F and MD1610F cover the use of Dalargin in cases of chronic recurrent oral ulceration, and patent RU 2230549 covers its use for the treatment of allergic dermatosis. A publication by A.V. Dontsov [19] demonstrated the efficacy of Dalargin for cytokine profile correction in patients with ischaemic heart disease and metabolic syndrome. At the same time, the present invention, while claiming the efficacy of Dalargin in burn therapy, is fundamentally different from the cited works, which pertain to the treatment of other diseases.

To summarize the above, it can be concluded that Dalargin shows great promise from the standpoint of clinical development, and its multifaceted activity, which eliminates pathophysiological processes (oxidative stress, LPO, hyperinflammatory and nociceptive response) and stimulates reparative processes, is responsible for the undeniable advantages of this product as a topical treatment of skin wounds and burns.

The closest analogue of the claimed invention is D-Panthenol, a topical medication with wound- and burn-healing properties that contains per 1 g: as the active ingredient: dexpanthenol: 50 mg; excipients: Phenonip: 4 mg; lanolin: 160 mg; white beeswax: 50 mg; soft white paraffin: 210.8 mg; dimethicone: 5 mg; Lanette SX emulsifier: 20 mg; propylene glycol: 20 mg; butylhydroxyanisole: 0.1 mg; butylhydroxytoluene: 0.1 mg; decamethylcyclopentasiloxane: 20 mg; magnesium sulfate heptahydrate: 5 mg; protegin W: 295 mg; purified water: 160 mg (RLS, entry for D-Panthenol. Last updated by the manufacturer on 19/09/2017, https://www.rlsnet.ru/tn_index_id_12869.htm)

The active ingredient of this product is dexpanthenol, which works by stimulating skin regeneration, normalising cell metabolism and increasing the strength of collagen fibres. An obvious disadvantage of this medicinal product lies therefore in the fact that it does not involve other biological mechanisms that are activated in cases of skin injury and participate in wound healing.

### Disclosure of the invention

In view of the above, the goal of the invention is to create a burn and wound healing agent for topical application that acts on a variety of mechanisms contributing to the healing of wounds of different etiologies.

Technical results of the claimed invention are:
- the use of the hexapeptide tyrosyl-D-alanyl-glycyl-phenylalanyl-leucyl-arginine for stimulating tissue regeneration processes in the wound region, reducing the severity of productive inflammation after thermal injury and reducing oedema and hyperaemia.
- the pronounced wound and burn healing effect of the claimed substance as part of the composition for topical use, which results from its multifaceted activity involving elimination of pathophysiological processes (oxidative stress, LPO, hyperinflammatory and nociceptive response) and enhancement of reparative processes, ensuring undeniable advantages of this product in the local therapy of skin wounds and burns.

For achieving this goal, we propose an agent for use in the treatment of skin wounds or burns in the form of a dosage form for topical use containing, as the active hexapeptide tyrosyl-D-alanyl-glycyl-phenylalanyl-leucyl-arginine at 0.02 - 0.5 weight %, with a pharmaceutically acceptable excipient / pharmaceutically acceptable excipients making up the rest.

In a particular embodiment of the invention, the agent is a solution containing the hexapeptide tyrosyl-D-alanyl-glycyl-phenylalanyl-leucyl-arginine and a pharmaceutically acceptable excipient at the following ratio of the ingredients in weight %: hexapeptide tyrosyl-D-alanyl-glycyl-phenylalanyl-leucyl-arginine: 0.02 - 0.5 weight %, purified water up to 100 %.

In a particular embodiment of the invention, the agent is a gel containing the hexapeptide tyrosyl-D-alanyl-glycyl-phenylalanyl-leucyl-arginine and pharmaceutically acceptable excipients at the following ratio of the ingredients in weight %: hexapeptide tyrosyl-D-alanyl-glycyl-phenylalanyl-leucyl-arginine: 0.02 - 0.5 %, hydroxyethyl cellulose 2.5 - 3 %, potassium sorbate 2 - 3 %, purified water up to 100 %.

### Brief description of the drawings and other explanatory materials.

Table 1. Burn wound oedema score vs. time as a measure response to thermal injury, where ^{∗} marks statistically significant differences relative to the control group, ^{∗∗} marks statistically significant differences relative to the dexpanthenol group.
Table 2. Burn wound hyperaemia score vs. time, where ^{∗} marks statistically significant differences relative to the control group, ^{∗∗} marks statistically significant differences relative to the dexpanthenol group.
Table 3. Planimetric evaluation results (hexapeptide tyrosyl-D-alanyl-glycyl-phenylalanyl-leucyl-arginine in the form of a solution): burn area (in cm²), where ^{∗} marks statistically significant differences relative to the control group, ^{∗∗} marks statistically significant differences relative to the dexpanthenol group.
Table 4. Planimetric evaluation results (hexapeptide tyrosyl-D-alanyl-glycyl-phenylalanyl-leucyl-arginine in the form of a gel): burn area (in cm²), where ^{∗} marks statistically significant differences relative to the control group, ^{∗∗} marks statistically significant differences relative to the Bepanthen group.
Figure 1. Average healing rate of burn wounds (cm²/day), where I = control, II = Dexpanthenol, III = hexapeptide tyrosyl-D-alanyl-glycyl-phenylalanyl-leucyl-arginine (0.02 - 0.5 % solution).
Figure 2. Lymphocyte-neutrophil ratio vs. time after thermal injury, where the vertical axis represents the ratio, and the horizontal axis represents follow-up days; 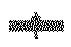 = control, 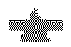 = Dexpanthenol, 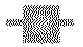 = hexapeptide tyrosyl-D-alanyl-glycyl-phenylalanyl-leucyl-arginine (0.02 - 0.5 % solution).
Figure 3. Effect on skin wound healing, where the horizontal axis represents follow-up days and the vertical axis represents the mean linear dimension of the wound; 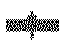 = control, 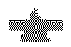 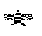 = Dexpanthenol, 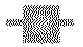 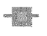 = hexapeptide tyrosyl-D-alanyl-glycyl-phenylalanyl-leucyl-arginine (0.02 - 0.5 % solution).
Figure 4. Average healing rate of burn wounds (cm²/day), where I = control, II = Bepanthen, III = hexapeptide tyrosyl-D-alanyl-glycyl-phenylalanyl-leucyl-arginine (0.02 % gel), IV = hexapeptide tyrosyl-D-alanyl-glycyl-phenylalanyl-leucyl-arginine (0.5 % gel)

### Embodiment of the invention

### Example 1. Production of the agent in the form of a topical solution for the treatment of skin wounds and burns

Production of the composition containing the hexapeptide tyrosyl-D-alanyl-glycyl-phenylalanyl-leucyl-arginine in the form of a solution for topical use includes: preparation of a solution of the hexapeptide tyrosyl-D-alanyl-glycyl-phenylalanyl-leucyl-arginine, vial filling, quality control of the solution.

Add the calculated quantity of dalargin (0.04 - 1.0 g) in 200 mL of purified water under mixing and mix until completely dissolved. Allow the resulting solution to pass through a sterile filter (pore size 0.22 µm) to produce the solution for topical use.

### Composition for topical use

| Name | Content in g | |
|---|---|---|
| Hexapeptide tyrosyl-D-alanyl-glycyl-phenylalanyl-leucyl-arginine | | 0.02-0.5 |
| Purified water | | up to 100 mL |

### Example 2. Production of the agent in the form of a topical gel for the treatment of skin wounds and burns

For the production of 150 mL of the claimed gel, take:
hexapeptide tyrosyl-D-alanyl-glycyl-phenylalanyl-leucyl-arginine: 0.04 - 0.75 g, purified water: 135 - 139 mL, hydroxyethyl cellulose: 4 - 4.5 g, potassium sorbate: 3 - 4 g.

Charge the reactor with a quantity of water required according to the manufacturing formula, turn on the mixing, add powdered components, mix until completely molten, then add the remaining components as required by the manufacturing formula. Continue mixing for 30 minutes until a homogeneous emulsion is obtained. Collect a test sample. If the values of test parameters correspond to Manufacturer's monograph, unload the unpacked finished product in an intermediate container and transfer it to the filling stage.

Composition of the gel containing the hexapeptide tyrosyl-D-alanyl-glycyl-phenylalanyl-leucyl-arginine

| Name | Content, weight % | |
|---|---|---|
| Hexapeptide tyrosyl-D-alanyl-glycyl-phenylalanyl-leucyl-arginine | | 0.02-0.5 |
| Hydroxyethyl starch | | 2.5-3 |
| Potassium sorbate | | 2-3 |
| Purified water | | up to 100 |

### Example 3. Results of an experiment to evaluate the efficacy of the hexapeptide tyrosyl-D-alanyl-glycyl-phenylalanyl-leucyl-arginine in a burn model in rats.

The efficacy of topical administration of the hexapeptide tyrosyl-D-alanyl-glycyl-phenylalanyl-leucyl-arginine in the therapy of thermal burns was evaluated on models of II-IIIA degree burns in 30 outbred male rats. The animals were kept in a vivarium (ambient temperature 19 - 23 °C) according to Good Laboratory Practices of the Eurasian Economic Union and the applicable guidelines [20].

Flat-bottom glass containers of the appropriate diameter filled with liquid paraffin to 2/3 of their volume were used as the thermal agent. The containers were heated on a water bath before application to the skin. The animals were randomised to three groups (n = 10 in each group): the control group and two experimental groups. Another tissue repair stimulator, dexpanthenol (5 % topical ointment), was used as the comparator. All the rats received burns with a standardised area of ≈40 cm².

Within 20 min. after inflicting the thermal injury, control group animals were treated by applying an aseptic dressing followed by therapy with Iodopiron and Levomecol (group I); animals in the experimental groups additionally received dexpanthenol (group II) or 0.02 - 0.5 % solution of the hexapeptide tyrosyl-D-alanyl-glycyl-phenylalanyl-leucyl-arginine (group III consisting of 5 animals that received 0.02 % solution and 5 animals that received 0.5 % solution).

The experiment involved evaluating the general condition of the animals and performing gross examination to evaluate the severity of tissue oedema in the burn region, hyperaemia, infiltration, appearance of wound discharge and wound \epitheliasation. These signs were scored as follows: maximum severity of a sign corresponded to a score of 3 points, and absence of a sign to a score of 0. Objective assessment of the healing process was based on planimetric measurements. All the observations were performed on days 1, 3, 5, 7 and 10 of the experiment.

The healing rate was determined using the formula V = (Sl-S2)/t, where S1 = wound area during the previous measurement, S2 = wound area during the next measurement, t = number of days between the previous and the next measurement.

The experiment also included an evaluation of the white blood cell population as a ratio of the total number of lymphocytes to segmented cells in venous blood.

Results. On day 1 of the experiment, the general condition of all the test animals was evaluated as moderate, which was due to the extent of the inflicted thermal injury. The animals in the cages exhibited little movement, slept most of the time and weakly responded to external stimuli.

By day 3 their activity and mobility increased, the animals exhibited a defensive response when taken out of the cages, developed appetite, etc. No intergroup differences in the general condition of the animals were observed. Table 1 lists oedema scores of the burn wound and adjacent tissues. Analysis of the results indicates that the initial oedema as a response to thermal injury and the time of its disappearance were significantly less in the group receiving the hexapeptide tyrosyl-D-alanyl-glycyl-phenylalanyl-leucyl-arginine both relative to the control group and to the Dexpanthenol group.

Similar dynamics was also observed in the case of hyperaemia (Table 2). The treatments reduced skin erythema already on day 1. Meanwhile, the hexapeptide tyrosyl-D-alanyl-glycyl-phenylalanyl-leucyl-arginine was significantly superior to Dexpanthenol in reducing hyperaemia.

Planimetry demonstrated pronounced statistically significant positive dynamics of burn wound healing in group III animals (who received the hexapeptide tyrosyl-D-alanyl-glycyl-phenylalanyl-leucyl-arginine). It was characterised by significant reduction of burn area already on day 3 of the experiment, reduction of burn depth and, consequently, more favourable course of the wound healing process. Results obtained in groups II and III also demonstrated the superiority of the hexapeptide tyrosyl-D-alanyl-glycyl-phenylalanyl-leucyl-arginine in reducing the area of burn injury (Table 3).

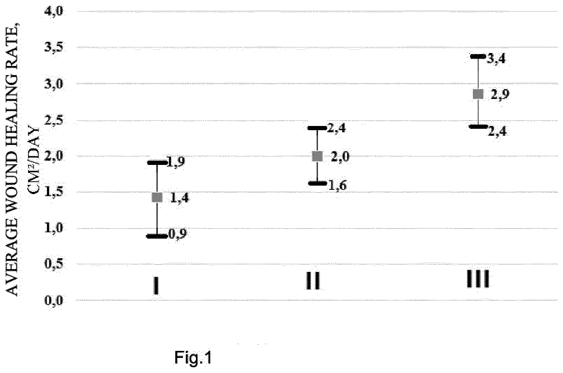

The average healing rate is shown in Fig. 1. Statistically significant differences were only detected between the control group and the group receiving the hexapeptide tyrosyl-D-alanyl-glycyl-phenylalanyl-leucyl-arginine.

Change of the lymphocyte-neutrophil ratio over time. Considering that one distinguishing trait of rats as a biological model is the predominance of monocytes in the white blood cell population of the peripheral blood (with lymphocytes normally making up 65 - 77 % of all white blood cells in healthy animals), the change in white blood cell profiles in the study groups was investigated by evaluating the lymphocyte-neutrophil ratio (the ratio of lymphocytes to segmented cells).

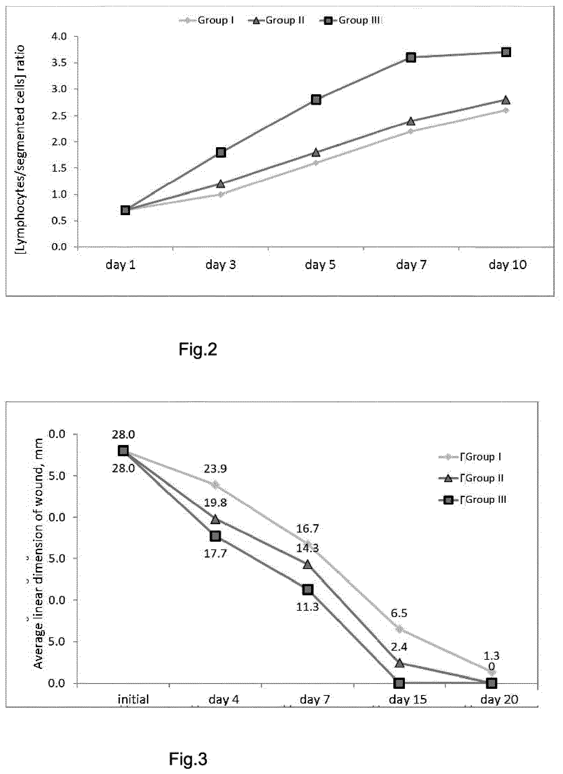

Analysis demonstrated that on day 1 the lymphocyte-neutrophil ratio substantially declined, indicating the expected response to thermal injury. Changes of the lymphocyte-neutrophil ratio over time, which are shown in Fig. 2, exhibit statistically significant differences between group I (controls) and group III (hexapeptide tyrosyl-D-alanyl-glycyl-phenylalanyl-leucyl-arginine). In group III this parameter returned to normal already on day 7, while in the other groups it continued to exhibit an upward trend.

To summarize, topical administration of the hexapeptide tyrosyl-D-alanyl-glycyl-phenylalanyl-leucyl-arginine immediately after thermal injury allows to reduce oedema and hyperaemia. It also demonstrates pronounced efficacy in terms of wound surface reduction, average wound healing rate and dynamics of the lymphocyte-neutrophil ratio.

The results obtained in the preclinical experiment provide evidence of the high efficacy of the hexapeptide tyrosyl-D-alanyl-glycyl-phenylalanyl-leucyl-arginine both as a first-aid treatment and in the subsequent therapy of moderate surface burns. Additionally, the results justify the statement that the hexapeptide tyrosyl-D-alanyl-glycyl-phenylalanyl-leucyl-arginine can also be used for wound bed preparation in cases of deep burns for subsequent skin transplantation and in the treatment of trophic ulcers in weakened patients with a low regenerative potential.

### Example 4. Results of an experiment to evaluate the efficacy of the hexapeptide tyrosyl-D-alanyl-glycyl-phenylalanyl-leucyl-arginine in a planar wound model in rats.

The study was performed in white male Wistar rats. All the animals were randomized to three groups of 13 rats each: (I) control group (untreated), (II) comparator group (Bepanthen topical cream with 5 % dexpanthenol) and (III) experimental group (hexapeptide tyrosyl-D-alanyl-glycyl-phenylalanyl-leucyl-arginine). The experimental group consisted of 5 animals that received 0.02 % solution and 5 animals that received 0.5 % solution. Average statistics for this group are provided in the text of the application.

Linear wounds were modelled by an approximately 4-cm-long longitudinal incision in the skin and subcutaneous fat along the midline of the back. After making the incision, the edges of the wound were brought together, and three sutures were applied at an equal distance. The animals received the study drugs starting on the day of the operation; the control group received a placebo. In each group 3 rats were intended for a tensometric study, which required euthanasia on day 7 of the experiment. Samples for a histological evaluation were collected immediately after complete healing (on days 25 - 26).

The study drugs were applied once daily, every day, for 20 days. The wound healing effect was evaluated based on the clinical course (presence of purulent discharge, dynamics of wound edge attachment) on days 4, 7, 15 and 20 of the experiment.

Fig. 3 presents results of visual inspection that clearly demonstrate the more rapid healing of the linear wound in the experimental group (hexapeptide tyrosyl-D-alanyl-glycyl-phenylalanyl-leucyl-arginine) relative to the control group and active comparator group.

Healing days 15-20, which correspond to the third phase of healing, are known to be marked by epitheliasation and scar formation. However, healing in the control group was not complete even on day 20 of observation, the scab remained, and complete epitheliasation in this group occurred only on days 25-26.

Based on microscopic examination results, collagen fibres in the intact zone in the control groups were loosely arranged and thick, whereas in the wound healing region collagen fibres were much thinner, and a large number of capillaries remained, i.e. the scar exhibited multiple signs of immaturity. Additionally, epithelial regeneration in the wound edge consolidation zone was not complete. Focal and diffuse inflammatory infiltrates were observed in the scar structure. Granulation tissue extended deep in to the dermis, almost to the boundary of subcutaneous fat.

In the group receiving the hexapeptide tyrosyl-D-alanyl-glycyl-phenylalanyl-leucyl-arginine, the scar ended at the depth of hair follicles; emptying of the blood vessels was observed, and fibrous components were dominant in scar tissue relative to cellular components, which indicates that the scar was at the concluding phase of development.

Intermediate results of histological evaluation (between the control group and the group receiving the hexapeptide tyrosyl-D-alanyl-glycyl-phenylalanyl-leucyl-arginine) were obtained in the group receiving therapy with Bepanthen cream.

Results of tensiometric evaluation demonstrated substantial (over than twofold) superiority of the hexapeptide tyrosyl-D-alanyl-glycyl-phenylalanyl-leucyl-arginine relative to the control group.

### Example 5. Results of an experiment to evaluate the efficacy of the hexapeptide tyrosyl-D-alanyl-glycyl-phenylalanyl-leucyl-arginine in the form of 0.02% and 0.5% gel in a burn model in rats.

The efficacy of topical administration of the hexapeptide tyrosyl-D-alanyl-glycyl-phenylalanyl-leucyl-arginine in the therapy of thermal burns was evaluated on models of II-IIIA degree burns in 40 outbred male rats. The animals were kept in a vivarium according to Good Laboratory Practices of the Eurasian Economic Union and the applicable guidelines [20]. Flat-bottom glass containers of the appropriate diameter filled with liquid paraffin to 2/3 of their volume played the role of the thermal agent. The animals were randomised to four groups (n = 10 in each group): group I was the control group, and the other three were experimental groups. A popular tissue repair product, Bepanthen (topical cream with 5 % dexpanthenol), was used as the comparator. Burn area was standardised at ≈40 cm².

An aseptic dressing applied within the first 20 minutes after the burn, followed by therapy with Iodopiron and Levomecol, was used as the control. Group II was treated with Bepanthen. Group III received the hexapeptide tyrosyl-D-alanyl-glycyl-phenylalanyl-leucyl-arginine as 0.02% topical gel, while group IV received the hexapeptide tyrosyl-D-alanyl-glycyl-phenylalanyl-leucyl-arginine as 0.5% topical gel.

In addition to monitoring the general condition of the animals on days 1, 3, 5, 7 and 10 of the experiment, the healing process was evaluated planimetrically. The healing rate was determined using the formula V = (Sl-S2)/t, where S1 = wound area during the previous measurement, S2 = wound area during the next measurement, t = number of days between the previous and the next measurement.

Results. On day 1 of the experiment the general condition of all the test animals was moderate. Their condition improved and activity increased by day 3 of the experiment. No differences in the general condition of the animals between the study groups were observed at this stage.

Planimetric findings are presented in Table 4. There was a pronounced statistically significant positive dynamics of burn wound healing in groups III and IV (hexapeptide tyrosyl-D-alanyl-glycyl-phenylalanyl-leucyl-arginine 0.02% and 0.5% respectively). It was characterised by significant (both relative to the control group and the Bepanthen group) reduction of burn area already on day 3 of the experiment, reduction of burn area and depth and, consequently, more favourable course of the wound healing process. No statistically significant differences were detected between groups III and IV, although group IV demonstrated numerically greater reduction of burn area relative to group III (Table 4).

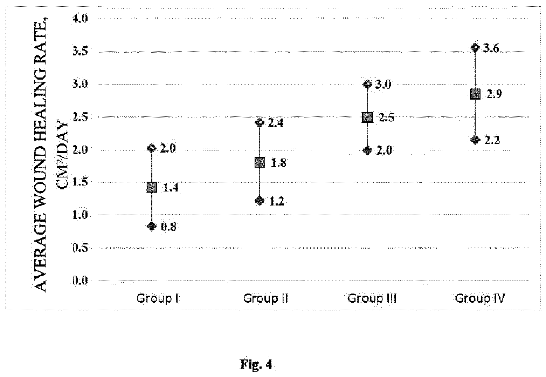

The average healing rate is shown in Fig. 4. Statistically significant differences were only detected between the control group (I) and the groups receiving the hexapeptide tyrosyl-D-alanyl-glycyl-phenylalanyl-leucyl-arginine in gel form (both 0.02 % and 0.5 %). Despite the fact that wound healing parameters in group IV were better, no significant differences relative to group III were detected owing to the small sample size in this study. The results confirm pronounced efficacy of the hexapeptide tyrosyl-D-alanyl-glycyl-phenylalanyl-leucyl-arginine as 0.02 % or 0.5 % gel with respect to reducing burn surface area and improving the average healing rate of burn wounds.

The results obtained in the preclinical experiment provide evidence of the high efficacy of the hexapeptide tyrosyl-D-alanyl-glycyl-phenylalanyl-leucyl-arginine both as a first-aid treatment and in the subsequent therapy of moderate surface burns. The results justify the statement that the hexapeptide tyrosyl-D-alanyl-glycyl-phenylalanyl-leucyl-arginine can be used in the topical gel or topical solution dosage forms for wound bed preparation in cases of deep burns for subsequent skin transplantation and in the treatment of trophic ulcers in patients with a low regenerative potential. The therapeutic range of concentrations for the hexapeptide tyrosyl-D-alanyl-glycyl-phenylalanyl-leucyl-arginine in the topical solution or topical gel dosage forms is 0.02 - 0.5 %.

### References

[1] World Health Organisation Fact Sheet, August 2017, URL: http://www.who.int/mediacentre/factsheets/fs3 65/ru.
[2] A.A. Alekseyev. Modern methods of burn and burn disease treatment (Sovremennyye metody lecheniya ozhogov i ozhogovoy bolezni) // Kombustiologiya (e-version). - 1999. - Nº. 1.
[3] A.N. Zokhirov et al. Effect of Dalargin, a Synthetic Analogue of Opioid Peptides, on the Anti-oxidant Status of Dogs // Vestnik Kurskoy Gosudarstvennoy Selskokhozyaystvennoy Akademii. - 2016. - Nº. 9.
[4] S.P. Lvova, T.F. Gorbunova, E.M. Abayeva. Effect of Hypothermia and Dalargin on Lipid Peroxidation in Rat Tissues (Vliyaniye gipotermii i dalargina na perekisnoye okisleniye lipidov v tkanyakh krys) // Voprosy Meditsinskoy Khimii. - 1993. - Vol. 39. - Nº.3. - p. 21-24.
[5] K.V. Zhmerenetski. Microcirculation and the Effects on it of Pharmaceutical Drugs of Different Classes in Cardiovascular Diseases (Mikrotsirkulyuatsiya i vliyaniye na neyo lekarstvennykh preparatov raznykh klassov pri serdechnososudistykh zabolevaniyakh). Dr. Med. Sci. Dissertation. - 2013.
[6] I.B. Zabolotskikh, S.V. Chuprin, A.N. Kurzanov. Dose-dependent Effects of Dalargin in Anaesthesiology and Intensive Care (Dozozavisimiye effekty dalargina v anesteziologii i intensivnoy terapii) // Vestnik Intensivnoy Terapii. - 2002. - Nº. 4. - p. 75-79.
[7] A.B. Shekhter et al. Effect of the Opiopeptide Dalargin on Reparation Processes during Wound Healing (Vliyaniye opiopeptida dalargina na reparativniye protsessy pri zazhivlenii ran) // Byull. eksperimentalnoy biologii i meditsiny. - 1988. - Nº. 10. - p. 487-490.
[8] T.D. Pankova, S.S. Timoshin. Evidence of the Stimulating Effect of Dalargin on the Cell Division Process being Realised through Opioid Receptors (Dokazatelstva realizatsii stimuliruyushchego effekta dalargina na protsess kletochnogo deleniye cherez opiatniye retseptory) // Byul. eksper. biol. i med. - 1990. - Nº. 7. - p. 96-98.
[9] A.A. Noskov et al. Effect of Dalargin on skin regeneration after a burn in rats (Vliyaniye preparata dalargin na regeneratsiyu kozhi posle ozhoga u krys) // Aktualniye Voprosy Meditsiny v Sovremennykh Usloviyakh. - 2017. - p. 59-60.
[10] Instruction for use of the medicinal product for human use Dalargin-Deko (LP-004596, web portal of the State Registry of Medicinal Products https://grls.rosminzdrav.ru/, accessed 31/01/2018).
[11] S.S. Timoshin, S.A. Alekseyenko, A.A. Shtuka. Effect of Dalargin on the Repair Capability of the Gastroduodenal Mucosa in Duodenal Ulcer Patients (Vliyaniye dalargina na reparativnuyu sposobnost gastroduodenalnoy slizistoy obolochki u bolnykh yazvennoy boleznyu dvenadsatiperstnoy kishki) // Klinicheskaya Meditsina - 1991. - Vol. 69. - Nº. 3. - p. 75-77.
[12] S.A. Alekseyenko et al. Effect of Dalargin on the Repair Capability of the Gastrointestinal Mucosa in Various Gastrointestinal Diseases (Vliyaniye dalargina na reparativnuyu sposobnost slizistoy obolochki zheludochno-kishechnogo trakta pri razlichnykh gastroenterologicheskikh zabolevaniyakh) // Dalnevostochny Meditsinsky Zhurnal. - 2010. - Nº. 3.
[13] V.A. Vinogradov, V.M. Polonsky, V.G. Smagin. Effect of Dalargin on Repair Processes in the Mucosa of the Gastroduodenal Region (Vliyaniye dalargina na reparativniye protsessy v slizistoy obolochke gastro-duodenalnoy zony) // Byull VKNTs. - 1982. - Nº. 5. - p. 40-42.
[14] E.V. Maksakova. Dalargin in the Treatment of Corneal Trauma (Dalargin v lechenii travmaticheskikh povrezhdeniy rogovitsy) // Oftalmolog. Zhurnal - 2000. - Nº. 6. - p. 95-97.
[15] D.K. Das, N. Molik. Transformation of the Death Signal into the Survival Signal in Redox Signalling (Prevrashcheniye signala gibeli v signal vyzhivaniya pri redoks-signalizatsii) // Biokhimiya. - 2004. - Vol. 69 No. 1. p. 16-24.
[16] E.Yu. Zhivotova, O.A. Lebedko, S.S. Timoshin. Effect of Structural Analogues of Leu-Enkephalin on DNA Synthesis Processes and Free Radical Oxidation in the Gastric Mucosa of White Mice (Vliyaniye strukturnykh analogov ley-enkefalina na protsessy sinteza DNK i svobodnoradikalnoye okisleniye v slizistoy obolochke zheludka belykh krys) // Dalnevostochny Meditsinsky Zhurnal. - 2012. - Nº. 1.
[17] B.V. Balachevsky, A.N. Kurzanov, A.A. Slavinsky. Dalargin-induced Modulation of Functional-Metabolic Activity of Neutrophilic Leukocytes // Uspekhi Sovremennogo Yestestvoznaniya. - 2008. - Nº. 5.
[18] V.I. Dontsov et al. Active Forms of Oxygen as a System: Significance for Physiology, Pathology and Natural Ageing (Aktivniye formy kisloroda kak sistema: znacheniye v fiziologii, patologii i estestvennom starenii) // Trudy ISA RAN. - 2006. - Vol. 19. - p. 50-69.
[19] A.V. Dontsov. Efficacy of Dalargin for the Correction of Cytokine Profile in IHD and Metabolic Syndrome Patients (Effektivnost dalargina v korrektsii tsitokinovogo profilya u bolnykh IBS i metabolicheskim sindromom) // Kursky Nauchno-Praktichesky Vestnik Chelovek i Ego Zdorovye. - 2013. - Nº. 1.
[20] Guidelines on the Clinical Studies of Medicinal Products. Part one. / ed. A.N. Mironov. Moscow. Grif i K, 2012. - 944 p.

To the application: "Agent for the Treatment of Skin Wounds or Burns"

**Table 1**

| Group | Day after receiving a thermal injury | | | | |
|---|---|---|---|---|---|
| | 1st | 3rd | 5th | 7th | 10th |
| Group I (control) | 3.0±0.3 | 2.0±0.4 | 0.9±0.3 | 0.3±0.3 | 0.0±0.0 |
| Group II (Dexpanthenol) | 2.0±0.5^{∗} | 1.3±0.4 | 0.7±0.5 | 0.3±0.3 | 0.0±0.0 |
| group III (hexapeptide tyrosyl-D-alanyl-glycyl-phenylalanyl-leucyl-arginine) | 1.0±0.4^{∗∗} | 0.7±0.4^{∗} | 0.3±0.2^{*} | 0.0±0.0 | 0.0±0.0 |

**Table 2:**

| Group | Day after receiving a thermal injury | | | | |
|---|---|---|---|---|---|
| | 1st | 3rd | 5th | 7th | 10th |
| Group I (control) | 2-6±0.2 | 1.5±0.3 | 0.7±0.2 | 0.0±0.0 | 0.0±0.0 |
| Group II (Dexpanthenol) | 1.8±0.2^{∗} | 1.4±0.2 | 0.5±0.3 | 0.0±0.0 | 0.0±0.0 |
| group III (hexapeptide tyrosyl-D-alanyl-glycyl-phenylalanyl-leucyl-arginine) | 0.8±0.3^{∗∗} | 0.7±0.2^{∗∗} | 0.0±0.0^{∗∗} | 0.0±0.0 | 0.0±0.0 |

To the application: "Agent for the Treatment of Skin Wounds or Burns"

**Table 3:**

| Group | Day after receiving a thermal injury | | | | | |
|---|---|---|---|---|---|---|
| | 1st | 3rd | 5th | 7th | 10th | 14th |
| Group I (control) | 40±2 | 23±2 | 20±3 | 18±2 | 15±2 | 12±2 |
| Group II (Dexpanthenol) | 40±2 | 18±3 | 15±2^{∗} | 13±2^{∗} | 12±3 | 7±3 |
| group III (hexapeptide tyrosyl-D-alanyl-glycyl-phenylalanyl-leucyl-arginine) | 40±2 | 12±3^{∗∗} | 11±2 | 8±2^{∗∗} | 6±2^{∗∗} | 0±0^{∗} ∗ |

**Table 4**

| Group | Day after receiving a thermal injury | | | | | |
|---|---|---|---|---|---|---|
| | 1st | 3rd | 5th | 7th | 10th | 14th |
| Group I (control) | 40±2 | 24±3 | 22±3 | 18±3 | 16±4 | 12±3 |
| group II (Bepanthen) | 40±2 | 19±3 | 16±2^{∗} | 14±2 | 12±3 | 8±3 |
| group III (hexapeptide tyrosyl-D-alanyl-glycyl-phenylalanyl-leucyl-arginine 0.02% gel) | 40±2 | 14±3 | 12±3^{∗∗} | 9±2^{∗∗} | 7±2 | 2±2^{∗∗} |
| group III (hexapeptide tyrosyl-D-alanyl-glycyl-phenylalanyl-leucyi-arginine 0.5% gel) | 40±2 | 11±3^{∗∗} | 10±3^{∗∗} | 7±2^{∗∗} | 5±2^{∗∗} | 0±0^{∗∗} |

## Claims

1. Agent for use in the treatment of skin wounds or burns in the form of a dosage form for topical use containing the hexapeptide tyrosyl-D-alanyl-glycyl-phenylalanyl-leucyl-arginine at 0.02 - 0.5 weight % as the active ingredient, with a pharmaceutically acceptable excipient / pharmaceutically acceptable excipients making up the rest.

2. Agent for use according to claim 1, wherein the agent is a solution containing the hexapeptide tyrosyl-D-alanyl-glycyl-phenylalanyl-leucyl-arginine and a pharmaceutically acceptable excipient at the following ratio of the ingredients in weight %: hexapeptide tyrosyl-D-alanyl-glycyl-phenylalanyl-leucyl-arginine: 0.02 - 0.5 weight %, purified water up to 100 %.

3. Agent for use according to claim 1, wherein the agent is a gel containing the hexapeptide tyrosyl-D-alanyl-glycyl-phenylalanyl-leucyl-arginine and pharmaceutically acceptable excipients at the following ratio of the ingredients in weight %: hexapeptide tyrosyl-D-alanyl-glycyl-phenylalanyl-leucyl-arginine: 0.02 - 0.5 weight %, hydroxyethyl cellulose 2.5 - 3 %, potassium sorbate 2 - 3 %, purified water up to 100 %.

## Patentansprüche

1. Mittel zur Verwendung bei der Behandlung von Hautwunden oder Verbrennungen in Form einer Dosierungsform zur topischen Verwendung, enthaltend das Hexapeptid Tyrosyl-D-alanyl-glycyl-phenylalanyl-leucyl-arginin mit 0,02 - 0,5 Gew% als Wirkstoff mit einem pharmazeutisch annehmbaren Trägerstoff / pharmazeutisch annehmbaren Trägerstoffen, die den Rest darstellen.

2. Mittel zur Verwendung nach Anspruch 1, wobei das Mittel eine Lösung ist, enthaltend das Hexapeptid Tyrosyl-D-alanyl-glycyl-phenylalanyl-leucyl-arginin und einen pharmazeutisch annehmbaren Trägerstoff mit dem folgenden Verhältnis der Bestandteile in Gew%: Hexapeptid Tyrosyl-D-alanyl-glycyl-phenylalanyl-leucyl-arginin 0,02 - 0,5 Gew%, gereinigtes Wasser bis zu 100 %.

3. Mittel zur Verwendung nach Anspruch 1, wobei das Mittel ein Gel ist, enthaltend das Hexapeptid Tyrosyl-D-alanyl-glycyl-phenylalanyl-leucyl-arginin und pharmazeutisch annehmbare Trägerstoffe mit dem folgenden Verhältnis der Bestandteile in Gew%: Hexapeptid Tyrosyl-D-alanyl-glycyl-phenylalanyl-leucyl-arginin 0,02 - 0,5 Gew%, Hydroxyethylcellulose 2,5 - 3 %, Kaliumsorbat 2 - 3 %, gereinigtes Wasser bis zu 100 %.

## Revendications

1. Agent pour une utilisation dans le traitement de plaies ou brûlures cutanées sous la forme d'une forme posologique à usage topique contenant l'hexapeptide tyrosyl-D-alanyl-glycyl-phénylalanyl-leucyl-arginine à raison de 0,02 à 0,5 % en poids en tant que principe actif, le reste étant constitué d'un ou plusieurs excipients pharmaceutiquement acceptables.

2. Agent pour une utilisation selon la revendication 1, lequel agent est une solution contenant l'hexapeptide tyrosyl-D-alanyl-glycyl-phénylalanyl-leucyl-arginine et un excipient pharmaceutiquement acceptable en le rapport suivant des ingrédients en % en poids : hexapeptide tyrosyl-D-alanyl-glycyl-phénylalanyl-leucyl-arginine : 0,02 à 0,5 % en poids, eau purifiée jusqu'à 100 %.

3. Agent pour une utilisation selon la revendication 1, lequel agent est un gel contenant l'hexapeptide tyrosyl-D-alanyl-glycyl-phénylalanyl-leucyl-arginine et des excipients pharmaceutiquement acceptables en le rapport suivant des ingrédients en % en poids : hexapeptide tyrosyl-D-alanyl-glycyl-phénylalanyl-leucyl-arginine : 0,02 à 0,5 % en poids, hydroxyéthylcellulose 2,5 à 3 %, sorbate de potassium 2 à 3 %, eau purifiée jusqu'à 100 %.
